# EUROPEAN PATENT SPECIFICATION

(11) **EP 1 499 377 B1**
(45) Date of publication and mention of the grant of the patent: **23.07.2008**
(21) Application number: 03730311.2
(22) Date of filing: 01.05.2003
(51) Int. Cl.: A61M 16/00, A61M 16/10, A61M 16/12

(54) **MEDICAL GAS RECIRCULATION SYSTEM**
RÜCKFÜHRUNGSSYSTEM FÜR MEDIZINISCHES GAS
SYSTEME DE RECIRCULATION DE GAZ MEDICAL

(30) Priority: 01.05.2002 GB 0210023
(43) Date of publication of application: 26.01.2005
(73) Proprietor: AIR PRODUCTS AND CHEMICALS, INC., Allentown, PA 18195-1501 (US)
(72) Inventor: Downie, Neil Alexander, Guildford, Surrey GU4 7EB (GB); Kerr, Stuart Alexander, Cheshire WA16 0DT (GB)
(74) Representative: Burford, Anthony Frederick
(86) International application number: PCT/GB2003/001875
(87) International publication number: WO 2003/092776

(56) References cited:
- EP-A- 0 745 405
- EP-A- 0 861 672
- SU-A- 1 188 638
- US-A- 4 903 693
- US-A- 6 131 571

## Description

The present invention relates to an apparatus and method for recirculating at least a binary gas mixture to a medical device such as a cardiac pulmonary bypass oxygenator or an artificial ventilator.

More particularly the invention relates to an apparatus and method for controlling the composition, pressure and flow rate of a recirculating gaseous composition to a medical device, particularly to a cardiopulmonary bypass oxygenator, and recycling the gaseous composition.

Medical devices such as cardiopulmonary bypass oxygenators and artificial ventilators or respirators require a reliable and constant source of gas for safe and reliable operation for use during the relevant medical procedures.

Usually, the gaseous compositions used for procedures with such devices are various air/oxygen or nitrogen/oxygen mixtures, although in some situations, these devices may be used for administering other active agents to a patient.

For example, it is common to use a respirator for administering an anaesthetic agent to anaesthetise a patient prior to undergoing certain surgical procedures. Xenon is known for use as an anaesthetic agent.

US-A-6131571 discloses a ventilation and/or anaesthesia delivery system having a recirculation circuit through which gas is circulated by a pump. Optionally, means are provided to introduce a liquid anaesthetic for vaporisation immediately downstream of the pump. After passage through a CO₂ absorber, bacterial filters, an optional heater or cooler and an optional manual breathing bag, the gas is delivered at a Y-piece connected to an endotracheal tube. The gas pressure at the Y-piece is controlled by a proportional flow control valve downstream of the Y-piece and operated in response to *inter alia* the gas composition at the distal end of the endotracheal tube. The size of an orifice in this valve is varied to alternately create (i) a pressure at the Y-piece that permits flow into the endotracheal tube and (ii) a pressure that permits gas flow from the tube into the circulation loop. In order to compensate for variations in gas flow between inhalation and exhalation, a volume reservoir is provided between the proportional control valve and the pump. Make-up gas is supplied via a pair of feed inlet valves in response to *inter alia* changes in the volume of recirculating gas in the reservoir. In order to provide for inhalation as well as exhalation, the gas feed to the pump is at subatmospheric pressure.

EP-A-0861672 discloses an inhalation apparatus having a recirculation circuit comprising a breathing bag, gas flow inducer; oxygen gas analyser; carbon dioxide gas analyser; temperature controller; gas flow switch; and absorbent filters. The gas flow switch provides for open cycle operation. Gas is supplied to a face mask by a gas inlet conduit controlled by an inhalation valve. Exhaled gas is returned to the recirculation circuit downstream of the inlet conduit via a microflora-removing device and an exhalation valve. A non-return valve is provided in the recirculation circuit between the inlet and outlet to the mask to prevent reversible of gas flow. Make-up gas is supplied by an inlet conduit upstream of the breathing bag. Oxygen is added via a valve responsive to signals from the oxygen and carbon dioxide gas analysers. Additional components are introduced into the feed conduit via respective valves but no detail of operation of these valves is provided. The gas flow inducer is stated to *reduce gasodynamic inhalation resistance by increasing the gas pressure* but no other details of its operation or construction are provided.

US-A-4989597 (Werner) discloses an apparatus for administration of at least two gases, particularly oxygen and xenon, to a patient via a respiration apparatus comprising a patient circuit and a drive circuit. The patient circuit, which enables rebreathing of the gas to make maximal use of valuable gases, is provided with fresh gas input to replace exhaled carbon dioxide with oxygen and to supplement the xenon concentration. The drive circuit and the patient circuit are in open communication and the concentration of each of the components in the patient circuit is independently monitored and controlled by addition of small quantities of one or other of the gases. The open communication between the patient circuit and the drive circuit results in an inherent equilibrium which it is stated allows the relative concentration of gases in the patient circuit to be more controllable. Xenon eventually accumulates in the drive circuit and can be recovered therefrom by supply to a recovery bottle once a certain concentration has been reached.

More recently, xenon has been identified as being useful in the treatment of neurointoxications, for example in WO-A-0053192. In particular, it is stated that xenon can reduce the release of neurotransmitters, particularly dopamine, which is caused by, for example, hypoxic situations such as an apoplexy or a craniocerebral trauma. It is stated (on page 5, lines 15-18 of WO-A-0053192) that use of the cardiopulmonary bypass machine can cause an unidentified neurointoxication, which significantly delays a patient's reconvalescence. According to WO-A-0053192, xenon may be administered by an inhalation method, or alternatively, may be added directly to a cardiopulmonary bypass machine. Further, WO-A-0108692 discloses the use of xenon as an NMDA antagonist to, for example, provide neuroprotection, relieve neuropathic pain or inhibit synaptic plasticity.

Under normal circumstances, cardiopulmonary bypass oxygenators are supplied with an oxygen/air or oxygen/nitrogen mixture on a once-through basis after which the spent gas (comprising the remaining oxygen, nitrogen and carbon dioxide flushed from the patient's blood) is vented to atmosphere. However, the use of xenon, or any other high value gas, in a cardiopulmonary bypass oxygenator would make this a very expensive procedure.

An apparatus and method for providing and recirculating gas to a medical device, such as a cardiopulmonary bypass oxygenator or an artificial ventilator, which also enables recovery of the high value gas is highly desirable, particularly when applied to a medical device used in an environment where space is at a premium.

Accordingly, in a first aspect of the invention there is provided an apparatus providing and circulating to a medical device a medical gas mixture comprising at least two components, said apparatus comprising:-
a main gas circuit for recirculating the medical gas and comprising:-
   a constant speed circulation pump for pumping gas through the main circuit and increasing the gas pressure from a lower pressure to a higher pressure,
   a pressure maintaining valve downstream of the pump and dividing the main circuit into a higher pressure section and a lower pressure section and thereby maintaining a constant pressure in the higher pressure section,
   a medical gas outlet in the higher pressure section,
   a spent gas inlet in the lower pressure section,
   a first feed gas supply inlet, preferably located in the higher pressure section,
   a second feed gas supply inlet, preferably located in the higher pressure section,
   a concentration determining means for measuring the concentration of at least one component of the recirculating medical gas mixture and generating a signal indicative of said concentration,
   circuit volume regulating means for varying the volume of the main circuit at a location in the lower pressure section for maintaining a predetermined gas flow to the pump and generating a signal indicative of said volume, and means for venting gas from the main circuit;
a first feed gas supply conduit for supply to the first feed gas inlet of a first feed gas of predetermined composition;
first feed gas supply flow control means for controlling the flow of first feed gas through the first gas supply conduit in response to the signal from the concentration determining means to maintain constant the medical gas composition at the pump inlet;
a second feed gas supply conduit for supply to the second feed gas inlet of a second feed gas of predetermined composition different from the first feed gas;
second feed gas supply flow control means for controlling the flow of second feed gas through the second gas supply conduit in response to the signal from the circuit volume regulating means to maintain constant the recirculating medical gas composition; and
a medical device supply circuit for connecting the medical device to the main circuit to receive a portion of the medical gas from the medical gas outlet thereof and to return spent gas to the spent gas inlet thereof and comprising:
   flow control means for controlling flow of the medical gas to the medical device and
   purification means for removing contaminant(s) from the spent gas.

In another aspect, the invention provides a medical device system comprising a medical device connected to the medical device supply circuit of an apparatus of the first aspect *supra.*

Preferably, the pressure maintaining valve is a spill valve; i.e. a valve which opens wider in response to increased pressure to pass more gas into the lower pressure section and thereby maintain the pressure in the higher pressure section. However, the valve could be a conventional pressure reduction valve.

Preferably, the circuit volume regulating means comprises expansion bellows and the means for generating a signal indicative of the volume thereof suitable is an infra-red level or, preferably, ultrasonic sensor for detecting the level of the expansion bellows in an expandable direction thereof.

The apparatus preferably operates at a pressure of up to about 250 mbarg (125 kPa) through the main circuit, more preferably up to about 150 mbarg (115 kPa) and may provide gas to the medical device at a pressure of up to about 100 mbarg (110 kPa), but preferably about 30 mbarg (103 kPa). The circulation pump may circulate gas through the circuit at a rate of up to about 80 litres per minute (l/min), preferably up to about 30 l/min, more preferably from about 15 to about 20 l/min and preferably supplies gas to the medical device at a rate of up to about 30 l/min, preferably up to about 10 l/min and still more preferably up to about 5 l/min.

Each of the first and second fed gas supply flow control means may be, for example, a valve or, preferably, a mass flow controller (MFC).

The concentration determining means measures the concentration of one or more individual components of the gas mixture.

If required, the gas concentration determining means and/or the circuit volume regulating means can provide a respective signal to alert an operator, for example, by way of an alarm, to the need to manually adjust the relevant supply flow control means.

Communication of the concentration determining means or circuit volume regulating means with the supply flow control means may be via an analog electrical circuit, on which the gain may be set as desired. For example, for control of a supply of a gas, such as oxygen, which is rapidly consumed and/or urgently required by the medical device, the analog circuit may have a high gain. Conversely, for control of a supply of a relatively slowly consumed gas, such as xenon, or inert, such as nitrogen, the analog circuit may have a low gain.

When the medical gas mixture is a binary gas mixture, typically the concentration of only one component is measured and the corresponding signal used to control the feed of that component to the respective feed inlet with the feed of the other component, or of a predetermined mixture of the two components, being controlled by the circuit volume regulating means signal.

When the medical gas mixture is a tertiary gas mixture, it is possible to operate in similar manner to a binary gas mixture using a separate feed for a first component and a mixed feed for the other two components optionally also with an amount of the first component. More usually, the three components at least primarily will be provided by three separate feeds. The concentration of two of the components can be measured and the individual concentration measurement signals used to control the corresponding respective feeds and the feed of the third component controlled by the circuit volume regulating means signal. Alternatively, the concentration of two of the components can be measured, one of the individual concentration measurement signals being used to control the corresponding feed and both the other concentration measurement signal and the circuit volume regulating means signal being used to control the feeds of the other two components.

For example, using a tertiary mixture of 40% oxygen, 20% xenon and 40% inert gas (usually nitrogen), feed control to maintain the gas composition can be achieved by using an oxygen concentration measurement signal to control the feed of oxygen and the circuit volume regulating means signal used to control the feed of a mixture of xenon, inactive gas and optionally oxygen. However, such a system does not allow full control in the presence of, for example air leaks or other events that affect only one of xenon and the inert gas and not the other. Accordingly, it is preferred to use three input gases, for example, (a) oxygen, (b) xenon or a mixture of xenon with a minor proportion of oxygen and (c) nitrogen or a mixture of nitrogen with a minor proportion of oxygen, and to control the flow rates of these using a tri-gas control system. A tri-gas control system can compensate not only for oxygen uptake by the medical device, but can also compensate, without error, for xenon and/or nitrogen uptake or emission by/from the device, dead volume filled with gas mixture or air, and leaks of air or other gases into or out of the system.

The tri-gas control system can be implemented by a straightforward proportional algorithm driven by two gas concentration signals and the system volume signal. For example, the oxygen can be added in an amount dependant on the difference between the measured and a predetermined oxygen concentrations; the xenon (or xenon/oxygen mixture) added in an amount dependant on the difference between the measured and a predetermined xenon concentrations; and the nitrogen (or nitrogen/oxygen mixture) added in an amount dependant on the extent to which the volume in the main circuit differs from a predetermined volume. However, in order to be assured of a stable control system where the different gas additions to not interact in a deleterious way, it is preferred to use both the difference in measured and predetermined xenon concentrations and between actual and predetermined circuit volumes to control the addition of both the xenon- and nitrogen- containing feeds. In particular, the xenon-containing feed is determined by the function YF, where: F = M' x (actual circuit volume - predetermined circuit volume), Y = M" x (actual xenon percentage concentration - predetermined xenon percentage concentration) and M' and M" are constant gain/multiplier factors, and the nitrogen-containing feed is determined by the function (A -Y)F, A is the maximum flow signal to the nitrogen-containing supply control means. Thus, if the gas supply control means are all MFCs having a 5V for 1 litre/min flow rate, the gain/multiplier factor for oxygen is 250, M' is 50 and M" is 35, (a) a measured oxygen concentration 2% low relative to the datum level, would result in the addition of 1 litre/min of oxygen; (b) a circuit volume 10% below the datum level would provide an F value of 5; and (c) a measured xenon concentration 2% low relative to the datum level would provide a Y value of 0.7. Thus under these conditions the YF signal woold be 3.5, resulting in the addition of 0.7 litre/min of the xenon-containing gas, and the (A-Y)F signal would be 1.5 (A = 5), resulting in the addition of 0.3 litre/min of the nitrogen containing gas.

Preferably the medical device is an artificial ventilator or, especially, a cardiopulmonary bypass oxygenator. The apparatus of the invention can selectively supply an artificial ventilator or a cardiopulmonary bypass oxygenator, whereby a patient can readily be ventilated immediately before and after cardiopulmonary bypass.

In a third aspect of the invention, there is provided a method of providing a medical device with a medical gas mixture comprising at least two components, said method comprising:-
recirculating the medical gas mixture in a main circuit having a higher pressure section maintained at constant pressure in series with a lower pressure section;
withdrawing a portion of the medical gas mixture from the higher pressure section and feeding said portion to the medical device;
removing contaminant(s) from the spent gas mixture from the medical device and returning the decontaminated spent gas to the lower pressure section;
replenishing components in the medical gas mixture by addition of feed gases to maintain the recirculating medical gas composition constant; and
varying the volume of the main gas circuit to maintain the gas flow therein

The method can be used for operating a medical device system in accordance with the second aspect of the present invention, however, operating of the medical device to treat the human or animal body by surgery or therapy or a diagnostic method practised on the human or animal body is not part of the claimed invention.

The apparatus and method of the invention can be used in a not claimed method for the extracorporeal treatment of blood by contacting blood with a recirculating medical gas mixture in a device provided with the medical gas mixture.

The gaseous composition for use in the present invention preferably contains at least one high value gas, which it would be beneficial to recover after use in the process. Such gases include the noble gases, especially xenon, krypton and neon or isotopes thereof, or stable isotopes of gases such as oxygen and carbon dioxide.

In a preferred embodiment, the gaseous composition comprises xenon, preferably in an amount of at least about 10% by volume, more preferably at least about 30%, still more preferably at least about 50% and still more preferably at least about 70% by volume. Most preferably, the gaseous composition comprises xenon in an amount of about 80% by volume.

The gaseous composition preferably also comprises oxygen and more preferably consists predominantly of xenon and oxygen. Most preferably, the gaseous composition comprises xenon and oxygen in a ratio of about 80% to about 20% by volume and usually will consist solely of xenon and oxygen.

The component gases may be replenished individually or in a mixture of gases, preferably a binary mixture, of known relative proportions.

Optionally, the gaseous composition may also comprise, for example, helium or nitrogen. Helium may be provide through a further supply flow conduit from, for example, a helium cylinder or a cylinder containing a helium/oxygen mixture. Nitrogen may be provided, for example, by admitting air to the circuit.

In a preferred embodiment of the invention, the medical device is a cardiopulmonary bypass oxygenator and the gaseous composition is a mixture predominantly of oxygen and xenon. Preferably the component gases are supplied from a first gaseous supply comprising oxygen and a second gaseous supply comprising xenon, which may be a xenon/oxygen mixture, for example in a ratio of about 80% to about 20%. Preferably the first gaseous supply is oxygen and the second gaseous supply is a xenon/oxygen mixture.

When oxygen is relatively quickly consumed, by a patient connected to the medical device, the oxygen concentration determining means, which may be, for example, an oxygen fuel cell sensor, preferably is connected to the first supply flow control means by a high gain electronic circuit enabling relatively rapid replenishment of oxygen to the circuit. For example, every 1% difference between the desired concentration and the detected concentration of oxygen may correspond to a flow through the oxygen (first) supply conduit of 1 litre per minute (l/min). Conversely, for controlling the concentration of xenon, which is relatively slowly consumed by a patient connected to the medical device, a low gain response may be more appropriate.

It is preferred that the concentration of xenon in a recirculating binary mixture with oxygen is determined with an ultrasonic gas analyser. Preferably, the ultrasonic gas analyser has an ultrahigh frequency ultrasonic transmitter, for example greater than 100 kHz. A suitable ultrasonic gas analyser is that described in EP-A-1499882.

The ultrasonic gas analyser may be used in combination with monitoring the recirculating volume to provide other information such as the concentration of contaminants in the circuit.

Similarly, comparison of the measured concentration of oxygen and xenon, in the recirculating gas, may provide information on the concentration of contaminants such as nitrogen or carbon dioxide.

When xenon or other high value gases are used, it is preferable to direct spent or recirculating gas that may from time to time be vented into a gas recovery space. Where the high value gas is provided from a supply in a fresh gas space in a container having an ullage space, the ullage space may provide the gas recovery space. Such a container can be as described in EP-A-9499828.

One or more of a carbon dioxide absorber, a carbon dioxide analyser and a pressure relief device can be provided downstream from the medical device when carbon dioxide is a waste product from that device.

The following is a description by way of example only and with reference to the accompanying drawings of presently preferred embodiments of the invention. In the drawings:
Figure 1 is a diagramatical representation of an apparatus according to one embodiment of the present invention for providing a xenon/oxygen mixture to a cardiopulmonary bypass oxygenator,
Figure 2 is a diagramatical representation of a ventilator circuit for introduction into the apparatus of Figure 1 to replace the cardiopulmonary bypass oxygenator;
Figure 3 is a diagramatical representation of another ventilator circuit for introduction into the apparatus of Figure 1 to replace the cardiopulmonary bypass oxygenator; and
Figure 4 is a diagramatical representation of an apparatus according to another embodiment of the present invention for selectively providing a xenon/oxygen mixture to a cardiopulmonary bypass oxygenator and an artificial ventilator.

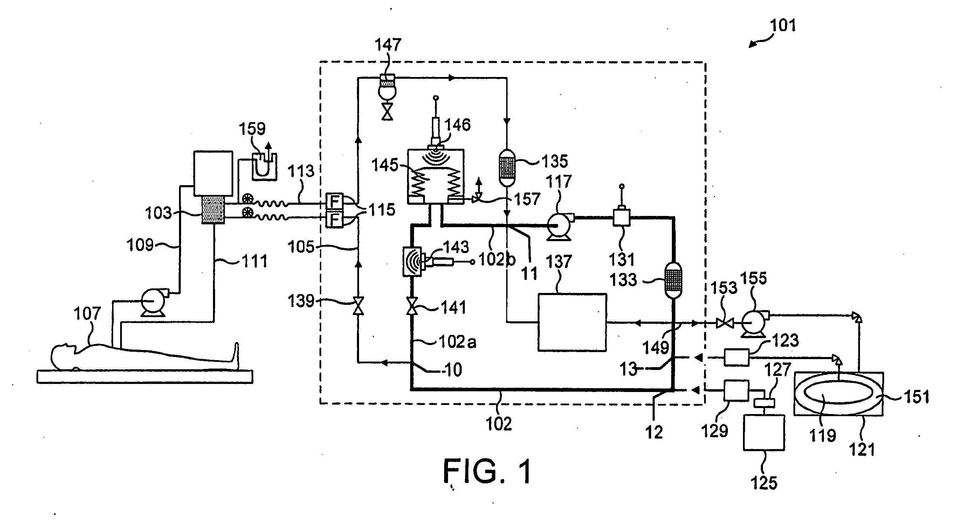

With reference to Figure 1, xenon/oxygen mixture in a ratio of 80% xenon to 20% oxygen is fed at inlet 13 into the main circuit 102 (a + b) of the apparatus (generally designated 101) from a xenon/oxygen supply in fresh gas space 119 of container 121 via xenon mass flow controller (MFC) 123.

The oxygen content of main circuit 102 is topped up at inlet 12 from oxygen cylinder 125 via regulator 127 and oxygen mass flow controller (MFC) 129.

One or more (preferably four) diaphragm pumps 117 pump the xenon/oxygen mixture around the circuit 102 at a rate of up to 20 litres per minute (I/min) at a pressure of up to 150 millibar gauge (115 kPa).

The gaseous composition is fed from outlet 10 to cardiopulmonary bypass (CPB) oxygenator 103 via medical device supply conduit 105, which is regulated by flow control valve 139, which may be set at a desired level by the operator.

CPB oxygenator 103, which is typically a membrane oxygenator, is fed unoxygenated blood from a patient 107 via unoxygenated blood conduit 109 and returned to the patient 107 via oxygenated blood conduit 111. Spent gas from the CPB oxygenator 103 is fed through spent gas return conduit 113 and then through water trap 147 and primary carbon dioxide absorber 135 to return to the main circuit section 102b upstream of pump(s) 117.

Gas passing through the spent gas return conduit 113 and medical device supply conduit 105 pass through respective bacterial filters 115 to protect the patient 107 from contamination from the apparatus 101 and vice versa.

In order to ensure that a constant flow of gas at the set pressure is supplied to the oxygenator 103 and thus available to the patient's blood, gas circulates through the main circuit 102 via pressure maintaining valve 141 downstream from the outlet to medical device supply conduit 105. Pressure maintaining valve 141 is a valve which allows gas flow only when the pressure exceeds a predetermined level, for example 30 mbarg (103 kPa) and accordingly maintains a constant pressure between the pumps 117 and the valve 141.

Downstream from the pressure maintaining valve 141, the gaseous composition is analysed for xenon content using ultrasonic xenon analyser 143 of the kind described in EP-A-1499882. In an alternative arrangement (not shown) the xenon analyser is located upstream of the pressure maintaining valve 141.

The gas is then fed via bellows 145, which expand to take up any additional volume of gas in the apparatus or contract to compensate for loss of volume in the apparatus, and receives the spent gas upstream of pump(s) 117.

The oxygen concentration in the main circuit 102 is monitored by an oxygen fuel cell sensor 131 that is shown situated in the main circuit section 102a downstream from pump(s) 117 but could be located downstream of the pressure maintenance valve 141. The gas is then fed through backup carbon dioxide absorber 133, which removes residual carbon dioxide from the recirculating gas. The carbon dioxide removed by absorbers 133 and 135 has entered via the oxygenator 103 after being flushed from the patient's blood. At least absorber 135 should be replaced with each use of the system.

Downstream from the backup carbon dioxide absorber 133, a small sample of gas is drawn from the main circuit 102 and fed to analyser unit 137 to be analysed for carbon dioxide, via an infra red gas analyser, to ensure that the carbon dioxide absorbers are working efficiently and for oxygen, via a paramagnetic gas analyser, as a backup to the oxygen fuel cell sensor 131. The sample is returned to the main circuit section 102b upstream from the pump(s) 117.

Recovery gas conduit 149 selectively feeds at least a portion of gas from the main circuit 102 at a point downstream from the backup carbon dioxide absorber 133 to the ullage space 151 of container 121, via recovery valve 153 and compressor 155. This container 121 is of the kind described in EP-A-1499828.

An atmospheric vent 157 from bellows 145 enables the gas within the apparatus to be vented to atmosphere if desired.

There is a U-tube relief device 159 on the spent gas return conduit 113 to protect the oxygenator 103 and patient 107 in the event of any back pressure from the apparatus 101.

Addition of fresh gas to the apparatus is controlled by an analog electronic circuit (not shown) between oxygen fuel cell sensor 131 and oxygen MFC 129 for fresh oxygen addition and by an analog electronic circuit between an ultrasonic level sensor 146 measuring the position of the bellows and the xenon MFC 123 for fresh xenon/oxygen mixture addition.

As well as monitoring the concentration of oxygen in the main circuit 102, oxygen fuel cell sensor 131 enables the oxygen concentration to be controlled. The operator may choose a set point on the sensor 131 corresponding to the desired oxygen concentration. When oxygen concentration measured by sensor 131 falls below the set point, oxygen MFC 129 is triggered to feed fresh oxygen into the main circuit 102 at a rate proportional to the difference between the oxygen level set point and the oxygen sensor 131 measurement via a high gain circuit connecting oxygen MFC 129 to sensor 131.

Typically, the high gain oxygen control circuit (not shown) will have a gain of 1, corresponding to an oxygen flow rate through oxygen MFC 129 and into the main circuit 102 of 1 l/min for every 1% difference between the oxygen set point and the measured oxygen level.

The xenon concentration of the main circuit is controlled by ultrasonic bellows level sensor 146. The operator may set the desired level on a potentiometer (not shown) connected to sensor 146, which corresponds to an expanded level of the bellows 145. This level corresponds to the volume in the system and, given that the oxygen concentration is known, to a desired concentration of xenon. When the sensor 146 detects that the bellows 145 has fallen below the desired level, xenon MFC 123 is triggered to feed fresh oxygen/xenon mixture into the main circuit 102 at a rate proportional to the difference between the potentiometer set point and the level measured by bellows sensor 146, via a low gain circuit (not shown) connecting sensor 146 to xenon MFC 123.

Typically, the xenon low gain circuit will have a gain of 0.1, corresponding to a flow of fresh xenon/oxygen mixture into the main circuit 102 of 0.1 I/min for every 1 % difference between the potentiometer setpoint and the level measured by bellows sensor 146.

The various sensor readings and flow rates are displayed on a monitoring unit (not shown).

In use, oxygen is consumed and replaced by carbon dioxide via the CPB oxygenator 103. The operator may select the flow rate to the oxygenator 103 by using flow control valve 139. This effectively controls the rate that carbon dioxide is flushed from patient's blood into the apparatus and hence provides some control as to the relative acidity or alkalinity of the patient 107.

Carbon dioxide is absorbed by primary carbon dioxide absorber 135 and the reduction in the oxygen level is detected by fuel cell sensor 131 triggering, via the high gain circuit, replenishment of oxygen levels under the control of oxygen MFC 129.

Xenon sensor 143 measures the xenon concentration in the main circuit 102. This reading may be compared to other readings to reach various conclusions. For example, if the oxygen concentration measured by oxygen fuel cell sensor 131 does not equal 100 minus the xenon concentration measured by xenon sensor 143, it is indicative of contamination, for example by carbon dioxide or nitrogen, and the operator may be alerted to vent the apparatus to atmosphere or recover the used gas. Alternatively, this may be done automatically at a preset level. The xenon sensor 143 is also used to monitor the xenon concentration predicted from the level of the bellows. Similarly, if these two readings do not agree, this may be indicative of too much carbon dioxide, nitrogen or oxygen. As a result, the operator may choose to vent to atmosphere or recover the used gas.

If the gas volume in the apparatus is increased, the level of bellows 145 increases. If the level of bellows 145 exceeds a preset level, gas is vented from the apparatus, again either manually or automatically, via atmospheric vent 157 and/or xenon recovery valve 153. Optionally, the sensor 146 may be connected to ultrasonic analyser 143 so that when the bellows 145 upper level is exceeded, vent 157 or valve 153 is selectively opened depending on the xenon content of the gas measured by analyser 143.

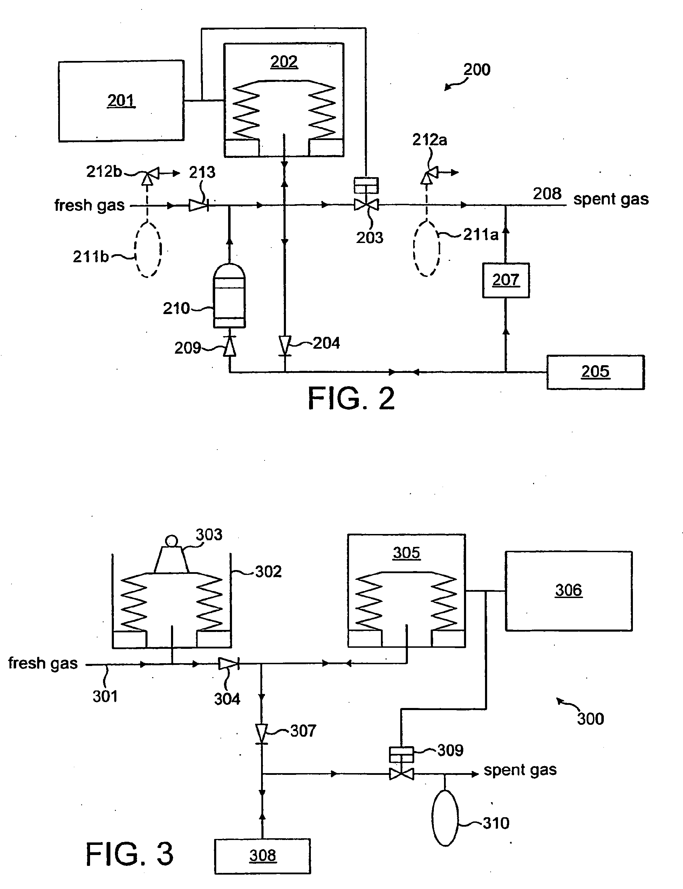

Referring now to Figure 2, a ventilator circuit generally designated 200 is connected at the filters 115 of the apparatus of Figure. 1 to replace the CPB circuit. Fresh gas passes through the outlet filter 115 (see Figure 1) into the ventilator circuit 200 via a check valve 213 to provide gas to the ventilator thereby maintaining the oxygen and xenon concentrations in the ventilator circuit 200 at the required levels.

The ventilator circuit 200 includes a conventional ventilator 201 of the kind providing a positive drive gas pressure (above atmospheric pressure) in pulses for a second or two, followed by a slightly longer period at atmospheric pressure. The period, cycle time and power of the drive gas pressure is set, in conventional manner, to match the needs of the patient 205.

When the ventilator drive pressure is positive, it pushes gas out of the bellows of a bellows assembly 202 via a control valve 203 and a check valve 204 into the lungs of the patient 205. Valve 203 is a pneumatically operated valve that is held closed by the positive ventilator drive pressure during the inflation of the patient's lungs. When the ventilator 201 proceeds to the atmospheric pressure part of its cycle, which allows the patient's lung to relax and deflate, exhaled gas (oxygen removed, carbon dioxide added) flows from the lungs via a check valve 209 to a soda-lime absorber canister 210. The canister 210 absorbs carbon dioxide from the exhaled gas and then allows it to flow back to refill the bellows of the bellows assembly 202. This gas may then be pumped back to the patient's lungs by the bellows during the next positive pressure pulse from the ventilator 201. The level of carbon dioxide in the gas from the patient's lungs is measured continuously by a CO₂ analyzer 207, which monitors both the end-tidal (peak) CO₂ level, which gives an indication of the patient's correct respiration, and the minimum CO₂ level, which gives an indication of exhaustion of the soda-lime 210.

When the ventilator 201 is in the atmospheric pressure part of its cycle, the valve 203 is open and, if the bellows inside the bellows assembly 202 has reached the top of its travel and the gas pressure becomes positive enough (a few millibar), gas may flow from the bellows into an optional bag 211a, past an optional pressure relief valve 212a and back to the gas recycling circuit 102 (see Figure 1) via an outlet 208 and filter 115 (see Figure 1). The bag 211 a and optional pressure relief valve 212a are needed if the tubing connecting the recycling circuit 102 to the ventilator circuit 200 are not large enough to assure correct operation of the bellows pressure relief via valve 203. In an alternative arrangement, the bag 211b and relief valve 212b are located upstream of the check valve 203.

Figure 3 shows an alternative ventilator circuit 300 for connection to gas main circuit 102 of Figure 1 in corresponding manner to the ventilator circuit 200 of Figure 2. It is specially designed to ensure that the patient 308 receives fresh gas from the main circuit 102 of Figure 1 and that the exhaled gas is not mixed with fresh gas but is fed back to the main circuit 102.

Fresh gas from the outlet filter 115 (see Figure 1) is fed to the ventilator circuit 300 at inlet 301. An optional feed bellows assembly 302 is connected downstream of the inlet and has a weight 303 to ensure that it runs at a small positive pressure, which is sufficient to feed gas through a check valve 304 to raise the bellows in a ventilator bellows assembly 305 when the drive gas pressure from ventilator 306 is atmospheric.

The ventilator 306 and bellows assembly 305 function in a similar way to that normally employed in prior art ventilator systems. Periodically, ventilator 306 applies positive (above atmospheric) gas pressure to the outside of the bellows in the bellows assembly 305, collapsing the bellows and forcing gas from inside the bellows through a check valve 307 to the lungs of the patient 308. The ventilator drive gas to the bellows is also applied to a pneumatically operated valve 309 to close it, so that all the gas from the bellows assembly 305 goes to the patient 308.

When the gas pressure from the ventilator 306 is relaxed back to atmospheric pressure, the bellows in bellows assembly 305 re-inflates with fresh gas from the inlet 301 and the feed bellows 302. The check valve 307 is biased with a spring or weight so that it only opens at a few millibar, assuring that 100% of fresh gas flows into the bellows assembly 305.

Simultaneously with the refill of the main bellows assembly 305 the patient's lungs relax, exhaling gas containing less oxygen and more carbon dioxide relative to fresh gas. The exhaled gas flows through pneumatically operated valve 309, which is now open to the gas return circuit (since the drive gas pressure is atmospheric). The gas return circuit may optionally include a variable gas volume comprising an additional bellows or flexible bag 310.

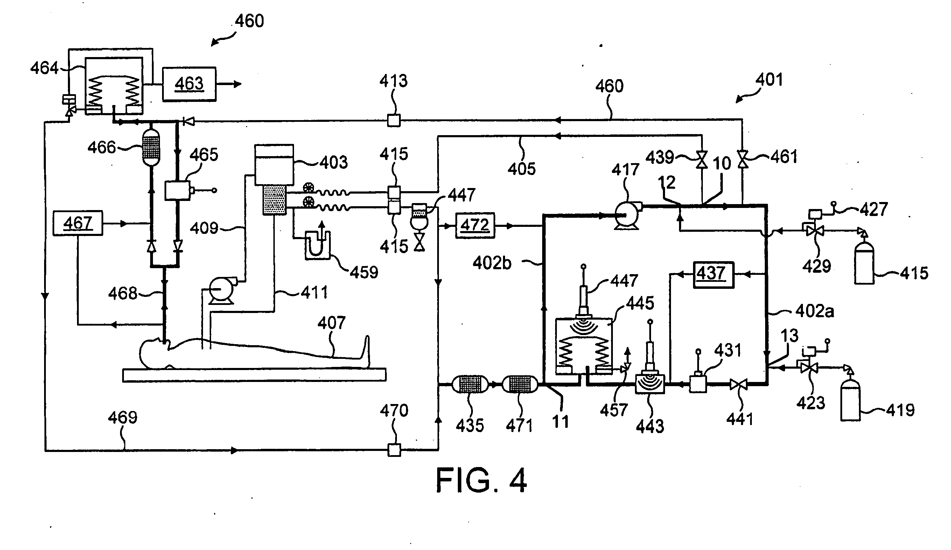

The embodiment of Figure 4 is similar to that of Figure 1 but provides for the selective supply of the xenon/oxygen mixture to an artificial ventilator and a CPB oxygenator so that xenon can be administered to the patient before, during and, if desired, after surgery. Many of the components of the embodiment of Figure 4 correspond to those of Figure 1 and accordingly have been identified by reference numerals in the 400 series corresponding to those in the 100 series used in Figure 1. Only the main differences between the two embodiments will be described.

In the embodiment of Figure 4, the xenon/oxygen mixture is provided by a conventional cylinder 419 instead of the ullage-space container 121 of Figure 1 and no provision is made for recovery of xenon. Further, the oxygen fuel cell sensor 431 is provided downstream, instead of upstream, of the pressure maintaining valve 441. A water adsorber 471 is provided immediately downstream of the primary carbon dioxide absorber 435 and a carbon dioxide analyzer 472 is provided to monitor the carbon dioxide content of the spent oxygenator gas.

A ventilator supply conduit 460 regulated by flow control valve 461 connects the main circuit section 402a downstream of the pumps 417 to an essentially conventional artificial ventilator assembly via a bacterial filter 413. The artificial ventilator assembly comprises the ventilator 463, bellows 464, oxygen fuel cell sensor 465, carbon dioxide absorber 466, carbon dioxide analyzer 467 and endotracheal tube 468 and operates generally as described with reference to Figures 2 and 3. The spent gas from the artificial ventilator assembly is returned to the main circuit via ventilator spent gas return conduit 469, including a bacterial filter 470, connected to the primary carbon dioxide adsorber 435.

Although illustrated and described herein with reference to certain specific embodiments, the present invention is nevertheless not intended to be limited to the details shown. Rather, various modifications may be made in the details within the scope of the following claims.

## Claims

1. An apparatus for providing and circulating to a medical device a medical gas mixture comprising at least two components, said apparatus comprising:- a main gas circuit (102a + 102b) for recirculating the medical gas mixture and comprising:-
a constant speed circulation pump (117) for pumping gas through the main circuit and increasing the gas pressure from a lower pressure to a higher pressure,
a pressure maintaining valve (141) downstream of the pump (117) and dividing the main circuit into a higher pressure section (102a) and a lower pressure section (102b) in order to maintain a constant pressure in the higher pressure section (102a),
a medical gas outlet (10) in the higher pressure section (102a),
a spent gas inlet (11) in the lower pressure section (1 02b),
a first feed gas supply inlet (12),
a second feed gas supply inlet (13) downstream of the medical gas outlet (10) and upstream of the pressure reduction valve (141), concentration determining means (131) for measuring the concentration of at least one component of the recirculating medical gas mixture and generating a signal indicative of said concentration,
circuit volume regulating means (145) for varying the volume of the main circuit at a location in the lower pressure section (102b) for maintaining a predetermined gas flow to the pump (117) and generating a signal indicative of said volume, and
means (157 + 149) for venting gas from the main circuit;a first feed gas supply conduit for supply to the first feed gas supply inlet (12) of a first feed gas (125) of predetermined composition;
first feed gas supply flow control means (127) for controlling the flow of first feed gas through the first gas supply conduit in response to the signal from the concentration determining means (131) to maintain constant the medical gas composition at the pump (117) inlet;
a second feed gas supply conduit for supply to the second feed gas supply inlet (13) of a second feed gas (119) of predetermined composition different from the first feed gas;
second feed gas supply flow control means (123) for controlling the flow of second feed gas through the second gas supply conduit in response to the signal from the circuit volume regulating means (145) to maintain constant the recirculating medical gas composition; and
a medical device supply circuit for connecting the medical device to the main circuit to receive a portion of the medical gas from the medical gas outlet (10) thereof and to return spent gas to the spent gas inlet (11) thereof and comprising:
flow control means (139) for controlling flow of the medical gas to the medical device and
purification means (135) for removing contaminant(s) from the spent gas.

2. An apparatus as claimed in Claim 1, wherein the feed gas supply inlets (12 & 13) are located in the higher pressure section (102a).

3. An apparatus as claimed in Claim 1 or Claim 2, wherein the pressure maintaining valve (141) is a spill valve.

4. An apparatus as claimed in any one of the preceding claims, wherein the circuit volume regulating means (145) comprises expansion bellows.

5. An apparatus as claimed in any one of the preceding claims, wherein the concentration determining means (131) comprises an analog electrical circuit for the signal thereof and the circuit volume regulating means (145) comprises an analog electrical circuit for the signal thereof which is of lower gain than that of the circuit for the signal of the concentration determining means (131), whereby the increase in flow rate of the first feed gas is quick relative to the increase in flow rate of the second feed gas.

6. An apparatus as claimed in any one of the preceding claims, wherein the concentration determining means (131) measures at least oxygen concentration.

7. An apparatus as claimed in any one of the preceding claims, wherein the concentration determining means (131) measures the concentration of at least two components and generates respective signals indicative of said concentrations and the apparatus further comprises:
a third feed gas supply inlet to the main gas circuit downstream of the medical gas outlet (10) and upstream of the pressure reduction valve (141);
a third feed gas supply conduit for supply to the third feed gas inlet of a third feed gas of predetermined composition different from the first and second feed gases; and
third feed gas supply flow control means for controlling the flow of third feed gas through the third gas supply conduit in response to the respective signal from the concentration determining means (131) to maintain constant the medical gas composition at the pump (117) inlet.

8. An apparatus as claimed in Claim 7, wherein both the second and third feed gas supply flow control means are responsive to a signal from the concentration determining means (131) and the signal from the circuit volume regulating means (145).

9. An apparatus as claimed in any one of the preceding claims, which further comprises an ultrasonic xenon analyser (147).

10. An apparatus as claimed in any one of the preceding claims, wherein the means (149) for venting gas from the main circuit comprising a gas recovery space (151) for storing at least a portion of the vented gas.

11. An apparatus as claimed in Claim 10, wherein the gas recovery space is an ullage space (151) of a container (121) providing one of the feed gases (119).

12. A medical device system comprising a medical device (103) connected to the medical device supply circuit of an apparatus as defined in any one of the preceding claims.

13. A system as claimed in Claim 12, wherein the medical device is an artificial ventilator (201).

14. A system as claimed in Claim 12, wherein the medical device is a cardiopulmonary bypass oxygenator (103).

15. A system as claimed in Claim 14, comprising both a cardiopulmonary bypass oxygenator (403) and an artificial ventilator (463) selectively connectable to the said medical device supply circuit.

16. A method of providing a medical device with a medical gas mixture comprising at least two components, said method comprising:-
recirculating the medical gas mixture in a main circuit having a higher pressure section maintained at constant pressure in series with a lower pressure section;
withdrawing a portion of the medical gas mixture from the higher pressure section and feeding said portion to the medical device;
removing contaminant(s) from the spent gas mixture from the medical device and returning the decontaminated spent gas to lower pressure section;
replenishing components in the medical gas mixture by addition of feed gases to maintain the recirculating medical gas composition constant; and
varying the volume of the main gas circuit to maintain the gas flow therein.

17. A method as claimed in Claim 16, wherein the medical device is a cardiopulmonary bypass oxygenator and/or an artificial ventilator.

18. A method as claimed in Claim 17, wherein medical gas mixture consists of oxygen and xenon.

19. A method as claimed in Claim 18, wherein the first feed gas is oxygen and the second feed gas is a mixture of xenon and oxygen.

20. A method as claimed in Claim 17, wherein medical gas mixture consists of oxygen, xenon and nitrogen.

21. A method as claimed in Claim 20, wherein the first feed gas is oxygen, the second feed gas is a mixture of xenon and oxygen and the third feed gas is air.

22. A method as claimed in Claim 20, wherein the first feed gas is oxygen, the second feed gas is xenon and the third feed gas is nitrogen and the concentrations of oxygen and nitrogen are measured.

23. A method as claimed in Claim 20, wherein the first feed gas is oxygen, the second feed gas is xenon and the third feed gas is nitrogen and the concentrations of oxygen and xenon are measured.

## Patentansprüche

1. Gerät zur Bereitstellung und Zirkulation eines medizinischen Gasgemisches, umfassend mindestens zwei Komponenten, in einer medizinischen Vorrichtung, wobei das Gerät umfasst:
einen Gashauptkreislauf (102a + 102b) zum Zirkulieren des medizinischen Gasgemisches und umfassend:
eine Zirkulationspumpe mit konstanter Geschwindigkeit (117) zum Pumpen von Gas durch den Hauptkreislauf und zum Erhöhen des Gasdruckes von einem niedrigeren Druck auf einen höheren Druck,
ein Ventil zur Aufrechterhaltung des Druckes (141) nach der Pumpe (117) und zur Aufteilung des Hauptkreislaufes in einen Abschnitt mit höherem Druck (102a) und einen Abschnitt mit niedrigerem Druck (102b), um einen konstanten Druck im Abschnitt mit höherem Druck (102a) aufrecht zu erhalten,
einen Auslass für medizinisches Gas (10) im Abschnitt mit höherem Druck (102a),
einen Einlass für verbrauchtes Gas (11) im Abschnitt mit niedrigerem Druck (102b),
einen Bereitstellungseinlass für ein erstes Zufuhrgas (12),
einen Bereitstellungseinlass für ein zweites Zufuhrgas (13) nach dem Auslass für medizinisches Gas (10) und vor dem Ventil zur Verringerung des Druckes (141),
Mittel zur Bestimmung einer Konzentration (131) zum Messen der Konzentration von mindestens einer Komponente des zirkulierenden medizinischen Gasgemisches und zum Erzeugen eines Signals, welches die Konzentration anzeigt,
Mittel zur Regulierung des Kreislaufvolumens (145) zum Variieren des Volumens des Hauptkreislaufs an einer Stelle im Abschnitt mit niedrigerem
Druck (102b) zum Aufrechterhalten eines vorher bestimmten Gasstroms zur Pumpe (117) und zum Erzeugen eines Signals, welches das Volumen anzeigt, und
Mittel (157 + 149) zur Entlüftung von Gas aus dem Hauptkreislauf;
eine Bereitstellungsleitung für ein erstes Zufuhrgas zum Bereitstellen eines ersten Zufuhrgases (125) mit vorher bestimmter Zusammensetzung am Bereitstellungseinlass für ein erstes Zufuhrgas (12);
Mittel zur Steuerung des Bereitstellungsstroms eines ersten Zufuhrgases (127) zum Steuern des Stroms eines ersten Zufuhrgases durch die Bereitstellungsleitung eines ersten Gases in Antwort auf das Signal des Mittels zur Bestimmung einer Konzentration (131), um die medizinische Gaszusammensetzung am Einlass der Pumpe (117) konstant zu halten;
eine Bereitstellungsleitung für ein zweites Zufuhrgas zum Bereitstellen eines zweiten Zufuhrgases (119) mit vorher bestimmter Zusammensetzung, unterschiedlich zum ersten Zufuhrgas, am Bereitstellungseinlass für ein zweites Zufuhrgas (13);
Mittel zur Steuerung des Bereitstellungsstroms eines zweiten Zufuhrgases (123) zum Steuern des Stroms eines zweiten Zufuhrgases durch die Bereitstellungsleitung eines zweiten Gases in Antwort auf das Signal des Mittels zur Regulierung des Kreislaufvolumens (145), um die Zusammensetzung des zirkulierenden medizinischen Gases konstant zu halten; und
einen Bereitstellungskreislauf der medizinischen Vorrichtung zum Verbinden der medizinischen Vorrichtung mit dem Hauptkreislauf, um einen Teil des medizinischen Gases vom Auslass für medizinisches Gas (10) davon zu erhalten und um verbrauchtes Gas zum Einlass für verbrauchtes Gas (11) davon rückzuführen, und umfassend:
Mittel zur Steuerung des Stroms (139) zum Steuern des Stroms des medizinischen Gases zur medizinischen Vorrichtung und
Reinigungsmittel (135) zur Entfernung von Verunreinigung(en) aus dem verbrauchten Gas.

2. Gerät wie in Anspruch 1 beansprucht, wobei die Bereitstellungseinlässe für Zufuhrgas (12 und 13) im Abschnitt mit höherem Druck (102a) lokalisiert sind.

3. Gerät wie in Anspruch 1 oder Anspruch 2 beansprucht, wobei das Ventil zum Aufrechterhalten des Druckes (141) ein Überstromventil ist,

4. Gerät wie in einem der vorhergehenden Ansprüche beansprucht, wobei das Mittel zur Regulierung des Kreislaufvolumens (145) Faltenbalge umfasst.

5. Gerät wie in einem der vorhergehenden Ansprüche beansprucht, wobei das Mittel zur Bestimmung einer Konzentration (131) eine analoge elektrische Schaltung für das Signal davon umfasst und das Mittel zur Regulierung des Kreislaufvolumens (145) eine analoge elektrische Schaltung für das Signal davon, welches langsamer zunimmt als das der Schaltung für das Signal des Mittels zur Bestimmung einer Konzentration (131), umfasst, wobei die Zunahme der Strömungsgeschwindigkeit des ersten Zufuhrgases, relativ zur Zunahme der Strömungsgeschwindigkeit des zweiten Zufuhrgases, schnell ist.

6. Gerät wie in einem der vorhergehenden Ansprüche beansprucht, wobei das Mittel zur Bestimmung einer Konzentration (131) mindestens eine Sauerstoffkonzentration misst.

7. Gerät wie in einem der vorhergehenden Ansprüche beansprucht, wobei das Mittel zur Bestimmung einer Konzentration (131) die Konzentration von mindestens zwei Komponenten misst und entsprechende Signale, welche die Konzentrationen anzeigen, erzeugt, und wobei das Gerät ferner umfasst:
einen Bereitstellungseinlass für ein drittes Zufuhrgas am Hauptgaskreislauf nach dem Auslass für medizinisches Gas (10) und vor dem Ventil zum Verringern des Drucks (141) und vor;
eine Bereitstellungsleitung für ein drittes Zufuhrgas zum Bereitstellen eines dritten Zufuhrgases mit vorher bestimmter Zusammensetzung, unterschiedlich von den ersten und zweiten Zufuhrgasen, am Einlass für ein drittes Zufuhrgas; und
Mittel zur Steuerung eines Bereitstellungsstroms eines dritten Zufuhrgases zum Steuern des Stroms eines dritten Zufuhrgases durch die Bereitstellungsleitung eines dritten Gases in Antwort auf das jeweilige Signal des Mittels zur Bestimmung einer Konzentration (131), um die Zusammensetzung des medizinischen Gases am Einlass der Pumpe (117) konstant zu halten.

8. Gerät wie in Anspruch 7 beansprucht, wobei sowohl das Mittel zur Steuerung eines Bereitstellungsstroms eines zweiten Zufuhrgases als auch das eines dritten Zufuhrgases auf ein Signal des Mittels zur Bestimmung einer Konzentration (131) und das Signal des Mittels zur Regulierung des Kreislaufvolumens (145) antworten.

9. Gerät wie in einem der vorhergehenden Ansprüche beansprucht, welches ferner eine Xenon-Ultraschallanalysevorrichtung (147) umfasst.

10. Gerät wie in einem der vorhergehenden Ansprüche beansprucht, wobei das Mittel (149) zur Entlüftung von Gas aus dem Hauptkreislauf einen Gasgewinnungsraum (151) zum Lagern von mindestens einem Teil des entlüfteten Gases umfasst.

11. Gerät wie in Anspruch 10 beansprucht, wobei der Gasgewinnungsraum ein Ausdehnungsraum (151) eines Behälters (121), welcher eines der Zufuhrgase (119) bereitstellt, ist.

12. Medizinisches Vorrichtungssystem, umfassend eine medizinische Vorrichtung (103), welche mit einem Bereitstellungskreislauf einer medizinischen Vorrichtung eines Geräts wie in einem der vorhergehenden Ansprüche definiert verbunden ist.

13. System wie in Anspruch 12 beansprucht, wobei die medizinische Vorrichtung ein künstliches Beatmungsgerät (201) ist.

14. System wie in Anspruch 12 beansprucht, wobei die medizinische Vorrichtung ein Herz-Lungen-Bypass-Oxygenator (103) ist.

15. System wie in Anspruch 14 beansprucht, umfassend sowohl einen Herz-Lungen-Bypass-Oxygenator (403) als auch ein künstliches Beatmungsgerät (463), welche selektiv an den Bereitstellungskreislauf der medizinischen Vorrichtung angeschlossen werden können.

16. Verfahren zur Bereitstellung einer medizinischen Vorrichtung mit einem medizinischen Gasgemisch, umfassend mindestens zwei Komponenten, wobei das Verfahren umfasst:
das Zirkulieren des medizinischen Gasgemisches in einem Hauptkreislauf mit einem Abschnitt mit höherem Druck, welcher bei konstantem Druck gehalten wird, in Reihe mit einem Abschnitt mit niedrigerem Druck;
das Abziehen eines Teils des medizinischen Gasgemisches aus dem Abschnitt mit höherem Druck und das Zuführen des Teils zu der medizinischen Vorrichtung;
das Entfernen von Verunreinigung(en) von dem verbrauchten Gasgemisch von der medizinischen Vorrichtung und das Rückführen des von Verunreinigungen befreiten verbrauchten Gases in den Abschnitt mit niedrigerem Druck;
das Nachfüllen von Komponenten in dem medizinischen Gasgemisch durch Zugabe von Zufuhrgasen, um die Zusammensetzung des zirkulierenden medizinischen Gases konstant zu halten; und
das Variieren des Volumens des Gashauptkreislaufs, um den Gasstrom darin aufrecht zu erhalten.

17. Verfahren wie in Anspruch 16 beansprucht, wobei die medizinische Vorrichtung ein Herz-Lungen-Bypass-Oxygenator und/oder ein künstliches Beatmungsgerät ist.

18. Verfahren wie in Anspruch 17 beansprucht, wobei das medizinische Gasgemisch aus Sauerstoff und Xenon besteht.

19. Verfahren wie in Anspruch 18 beansprucht, wobei das erste Zufuhrgas Sauerstoff ist und das zweite Zufuhrgas ein Gemisch von Xenon und Sauerstoff ist.

20. Verfahren wie in Anspruch 17 beansprucht, wobei das medizinische Gasgemisch aus Sauerstoff, Xenon und Stickstoff besteht.

21. Verfahren wie in Anspruch 20 beansprucht, wobei das erste Zufuhrgas Sauerstoff ist, das zweite Zufuhrgas ein Gemisch von Xenon und Sauerstoff ist und das dritte Zufuhrgas Luft ist.

22. Verfahren wie in Anspruch 20 beansprucht, wobei das erste Zufuhrgas Sauerstoff ist, das zweite Zufuhrgas Xenon ist und das dritte Zufuhrgas Stickstoff ist und die Konzentrationen von Sauerstoff und Stickstoff gemessen werden.

23. Verfahren wie in Anspruch 20 beansprucht, wobei das erste Zufuhrgas Sauerstoff ist, das zweite Zufuhrgas Xenon ist und das dritte Zufuhrgas Stickstoff ist und die Konzentrationen von Sauerstoff und Xenon gemessen werden.

## Revendications

1. Appareil pour fournir et faire circuler un mélange de gaz à usage médical à un dispositif médical comprenant au moins deux composants, ledit appareil comprenant:
un circuit principal de gaz (1 02a + 102b) pour recirculer le mélange de gaz à usage médical et comprenant:
une pompe de circulation à vitesse constante (117) pour pomper le gaz à travers le circuit principal et pour augmenter la pression de gaz d'une pression inférieure à une pression supérieure,
une soupape de maintien de pression (141) en aval de la pompe (117) et divisant le circuit principal en une section à pression supérieure (102a) et une section à pression inférieure (102b) afin de maintenir une pression constante dans la section à pression supérieure (102a),
une sortie (10) pour le gaz à usage médical dans la section à pression supérieure (102a),
une entrée (11) pour gaz usé dans la section à pression inférieure (102b),
une entrée d'alimentation en un premier gaz de charge (12),
une entrée d'alimentation en un deuxième gaz de charge (13) en aval de la sortie (10) pour gaz à usage médical et en amont de la soupape de réduction de pression (141),
un moyen de détermination de concentration (131) pour mesurer la concentration d'au moins un composant du mélange de gaz à usage médical de recirculation et pour générer un signal indiquant ladite concentration,
un moyen de régulation de volume du circuit (145) pour faire varier le volume du circuit principal à un emplacement dans la section à pression inférieure (102b) afin de maintenir un débit de gaz prédéterminé à la pompe (117) et générer un signal indiquant ledit volume, et
un moyen (157 + 149) pour évacuer le gaz depuis le circuit principal;
un conduit d'alimentation en un premier gaz de charge pour alimenter, avec un premier gaz de charge (125) ayant une composition prédéterminée, à l'entrée d'alimentation en un premier gaz de charge (12)
un moyen de commande du débit d'alimentation du premier gaz de charge (127) pour commander le débit du premier gaz de charge à travers le conduit d'alimentation en un premier gaz en réponse au signal depuis le moyen de détermination de concentration (131) pour maintenir la composition du gaz à usage médical constante à l'entrée de la pompe (117);
un conduit d'alimentation en un deuxième gaz de charge pour alimenter, avec un deuxième gaz de charge (119) d'une composition prédéterminée différente de celle du premier gaz de charge, à l'entrée d'alimentation en un deuxième gaz de charge (13);
un moyen de commande du débit d'alimentation du deuxième gaz de charge (123) pour commander le débit du deuxième gaz de charge à travers le conduit d'alimentation en un deuxième gaz en réponse au signal depuis le moyen de régulation de volume du circuit (145) pour maintenir la composition du gaz à usage médical de recirculation constante; et
un circuit d'alimentation du dispositif médical pour relier le dispositif médical au circuit principal afin de recevoir une partie du gaz à usage médical depuis la sortie de gaz à usage médical (10) correspondante et de renvoyer le gaz usé à l'entrée de gaz usé (11) correspondante et comprenant:
un moyen de commande de débit (139) pour commander le débit du gaz à usage médical vers le dispositif médical et
un moyen de purification (135) pour supprimer un (des) contaminant(s) du gaz usé.

2. Appareil comme revendiqué dans la revendication 1, dans lequel les entrées d'alimentation en gaz de charge (12 et 13) sont situées dans la section à pression supérieure (102a).

3. Appareil comme revendiqué dans la revendication 1 ou 2, dans lequel la soupape de maintien de pression (141) est une soupape de décharge.

4. Appareil comme revendiqué dans l'une quelconque des revendications précédentes, dans lequel le moyen de régulation de volume du circuit (145) comprend des joints de dilatation.

5. Appareil comme revendiqué dans l'une quelconque des revendications précédentes, dans lequel le moyen de détermination de concentration (131) comprend un circuit électrique analogique pour le signal correspondant et le moyen de régulation de volume du circuit (145) comprend un circuit électrique analogique pour le signal correspondant qui a un gain inférieur à celui du circuit pour le signal du moyen de détermination de concentration (131), l'augmentation du débit du premier gaz de charge est par ce moyen rapide par rapport à l'augmentation du débit du deuxième gaz de charge.

6. Appareil comme revendiqué dans l'une quelconque des revendications précédentes, dans lequel le moyen de détermination de concentration (131) mesure au moins la concentration en oxygène.

7. Appareil comme revendiqué dans l'une quelconque des revendications précédentes, dans lequel le moyen de détermination de concentration (131) mesure la concentration d'au moins deux composants et génère des signaux respectifs indiquant lesdites concentrations et l'appareil comprend en plus:
une entrée d'alimentation en un troisième gaz de charge au circuit principal de gaz en aval de la sortie (10) de gaz à usage médical et en amont de la soupape de réduction de pression (141) et en amont;
un conduit d'alimentation en un troisième gaz de charge pour alimenter à l'entrée d'alimentation en un troisième gaz de charge un troisième gaz de charge d'une composition prédéterminée différente de celle des premier et deuxième gaz de charge; et
un moyen de commande du débit de l'alimentation du troisième gaz de charge pour commander le débit du troisième gaz de charge à travers le conduit d'alimentation en un troisième gaz en réponse au signal respectif depuis le moyen de détermination de concentration (131) afin de maintenir la composition du gaz à usage médical constante à l'entrée de la pompe (117).

8. Appareil comme revendiqué dans la revendication 7, dans lequel le moyen de commande du débit de l'alimentation du deuxième gaz de charge et le moyen de commande du débit de l'alimentation du troisième gaz de charge sont tous deux sensibles à un signal du moyen de détermination de concentration (131) et au signal du moyen de régulation de volume du circuit (145).

9. Appareil comme revendiqué dans l'une quelconque des revendications précédentes, qui comprend en plus un analyseur à xénon ultrasonore (147).

10. Appareil comme revendiqué dans l'une quelconque des revendications précédentes, dans lequel le moyen (149) pour évacuer le gaz du circuit principal comprenant un espace de récupération de gaz (151) pour stocker au moins une partie du gaz évacué.

11. Appareil comme revendiqué dans la revendication 10, dans lequel l'espace de récupération de gaz est un espace mort (151) d'un récipient (121) procurant l'un des gaz de charge (119).

12. Système de dispositif médical comprenant un dispositif médical (103) relié au circuit d'alimentation du dispositif médical d'un appareil comme défini dans l'une quelconque des revendications précédentes.

13. Système comme revendiqué dans la revendication 12, dans lequel le dispositif médical est un insufflateur artificiel (201).

14. Système comme revendiqué dans la revendication 12, dans lequel le dispositif médical est un oxygénateur de circulation extracorporelle (103).

15. Système comme revendiqué dans la revendication 14, comprenant à la fois un oxygénateur de circulation extracorporelle (403) et un insufflateur artificiel (463) pouvant être reliés de manière sélective audit circuit d'alimentation du dispositif médical.

16. Procédé pour doter un dispositif médical d'un mélange de gaz à usage médical comprenant au moins deux composants, ledit procédé comprenant:
faire recirculer le mélange de gaz à usage médical dans un circuit principal ayant une section à pression supérieure maintenue à pression constante en série avec une section à pression inférieure à une pression constante;
retirer une partie du mélange de gaz à usage médical de la section à pression supérieure et alimenter ladite partie au dispositif médical;
supprimer un (des) contaminant(s) du mélange de gaz usé du diapositif médical et renvoyer le gaz usé désinfecté à la section à pression inférieure;
réapprovisionner des composants dans le mélange de gaz à usage médical en ajoutant des gaz de charge afin de maintenir la composition de gaz à usage médical de recirculation constante; et
faire varier le volume du circuit principal de gaz afin de maintenir le débit de gaz dans celui-ci.

17. Procédé comme revendiqué dans la revendication 16, dans lequel le dispositif médical est un oxygénateur de circulation extracorporelle et/ou un insufflateur artificiel.

18. Procédé comme revendiqué dans la revendication 17, dans lequel le mélange de gaz à usage médical consiste en de l'oxygène et du xénon.

19. Procédé comme revendiqué dans la revendication 18, dans lequel le premier gaz de charge est de l'oxygène et le deuxième gaz de charge est un mélange de xénon et d'oxygène.

20. Procédé comme revendiqué dans la revendication 17, dans lequel le mélange de gaz à usage médical consiste en de l'oxygène, du xénon et de l'azote.

21. Procédé comme revendiqué dans la revendication 20, dans lequel le premier gaz de charge est de l'oxygène, le deuxième gaz de charge est un mélange de xénon et d'oxygène et le troisième gaz de charge est de l'air.

22. Procédé comme revendiqué dans la revendication 20, dans lequel le premier gaz de charge est de l'oxygène, le deuxième gaz de charge est du xénon et le troisième gaz de charge est de l'azote et les concentrations d'oxygène et d'azote sont mesurées.

23. Procédé comme revendiqué dans la revendication 20, dans lequel le premier gaz de charge est de l'oxygène, le deuxième gaz de charge est du xénon et le troisième gaz de charge est de l'azote et les concentrations d'oxygène et de xénon sont mesurées.
